Europäisches Patentamt

European Patent Office

Office européen des brevets

(19)

(11) Veröffentlichungsnummer: **0 299 481**
**B1**

(12) **EUROPÄISCHE PATENTSCHRIFT**

(45) Veröffentlichungstag der Patentschrift:
20.06.90

(51) Int. Cl.⁵: **A61M 16/00**

(21) Anmeldenummer: 88111329.4

(22) Anmeldetag: 14.07.88

(54) Atemspendehilfsgerät.

(30) Priorität: 17.07.87 DE 8709867 U

(43) Veröffentlichungstag der Anmeldung:
18.01.89 Patentblatt 89/3

(45) Bekanntmachung des Hinweises auf die Patenterteilung:
20.06.90 Patentblatt 90/25

(84) Benannte Vertragsstaaten:
AT BE CH DE FR GB IT LI NL SE

(56) Entgegenhaltungen:
DE-B- 1 241 040
FR-A- 1 515 163
US-A- 3 013 554
US-A- 3 124 124
US-A- 4 535 765

Die Akte enthält technische Angaben, die nach dem Eingang der Anmeldung eingereicht wurden und die nicht in dieser Patentschrift enthalten sind.

(73) Patentinhaber: Reissmann, Hajo, Dr., Eggersallee 9,
D-2000 Hamburg 50(DE)

(72) Erfinder: Reissmann, Hajo, Dr., Eggersallee 9,
D-2000 Hamburg 50(DE)

(74) Vertreter: Glawe, Delfs, Moll & Partner Patentanwälte,
Postfach 26 01 62 Liebherrstrasse 20,
D-8000 München 26(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Atemspendehilfsgerät mit einem in die Mundhöhle zwischen Gaumen und Zunge einzusetzenden, etwa daumenlangen, vor dem Gaumensegel endenden Teil, einem damit starr verbundenen, außerhalb des Mundes zu liegen bestimmten Teil, mittels dessen die Winkelstellung des in der Mundhöhle befindlichen Teils vom Helfer eingestellt werden kann, und einem die beiden Teile voneinander abgrenzenden Lippenschild oder einer Mundabschlußkappe.

Atemspendehilfsgeräte dieser Art, die einen zwischen Gaumen und Zunge einzusetzenden, gekrümmten Tubus, einen Lippenschild und ein Beatmungsmundstück aufweisen, werden für die Atemspende in Notfallsituationen verwendet, um einen direkten Kontakt zwischen dem Helfer und dem Patienten und damit Infektionsgefahren zu vermeiden. Der Tubus dieser Geräte ist etwa daumenlang und reicht daher nicht in den Rachenraum des Patienten. Demgegenüber sind sogenannte Pharyngealtuben (beispielsweise Safar-Tubus und Guedel-Tubus) so lang ausgebildet, daß ihr Ende hinter der Zunge bis kurz vor den Kehlkopf reicht (Gorgaß, Ahnefeld: Der Rettungssanitäter, S. 130, 131). Dadurch wird sicher verhindert, daß der Atemweg durch Anlegen der Zunge gegen die hintere Rachenwand versperrt wird. Jedoch setzt die Einführung langer Pharyngealtuben in den Rachenraum einen besonders geschulten Helfer voraus, weil nicht nur bei unsachgemäßem Einsetzen des Tubus Verletzungen verursacht werden können, sondern auch bei korrekter Lage von Laien nicht zu erkennende und zu beherrschende Nebenwirkungen auftreten können. Bei den eingangs genannten, von Laien anzuwendenden Atemspendehilfsgeräten, auf die sich die Erfindung bezieht, ist deswegen der Tubus so kurz gestaltet, daß er nur im Mundraum des bewußtlosen Patienten zwischen Zunge und Gaumen liegt und noch vor dem Gaumensegel endet. Bei solchen Geräten werden zwar die Verletzungs- und Nebenwirkungsgefahren vermieden, aber es wird gleichzeitig auf den Vorteil einer sicheren Freihaltung der Atemwege durch Verwendung längerer Tuben verzichtet, da die Zunge zurücksinken und den Rachenraum versperren kann. Daran kann die Atemhilfe scheitern. Diese Gefahr, die auch bei Mundzu-Mund-Beatmung ohne Hilfsmittel auftritt, kann durch die bekannten Atemspendehilfsgeräte mit kurzen Tuben nicht beseitigt werden. Das gilt auch dann, wenn der Tubus so gekrümmt ist, daß er mit der gewölbten Seite über die Zunge fassen kann (Gebrauchsanweisung WM 16055 C "LIFEWAY" der Fa. Weinmann, Hamburg), weil die Zunge durch die Wölbung nicht sicher gehalten werden kann. Vielmehr besteht bei Verwendung solcher Geräte die Gefahr, daß der Helfer im Vertrauen auf ihre Funktionsfähigkeit den Verschluß des Atemwegs nicht erkennt.

Bei Pharyngealtuben ungeeigneter Länge besteht die Gefahr, daß die Endöffnung bei der Benutzung verschlossen wird, nämlich durch den Kehldeckel bei zu großer Länge bzw. durch den Zungengrund bei zu geringer Länge (Gorgaß, Ahnefeld a.a.O. Abb.88). Deshalb werden Pharyngealtuben am Ende zusätzlich mit Seitenöffnungen versehen (US-A 3 013 554, FR-A 1 375 232). Ähnliches gilt für solche Atemspendehilfsgeräte (DE-B 1 241 040), deren Tubus lediglich zwischen die Zähne des Patienten gesteckt wird, ohne weit in den Mund einzudringen, und dessen Endöffnung daher vom vorderen Zungenende versperrt werden kann. Grundsätzlich anders ist dies aber bei den der Erfindung zugrundeliegenden daumenlangen Tuben, weil für sie stets eine bestimmte Position zwischen Gaumen und Zunge vorgesehen ist, in der die endständige Öffnung frei bleibt. Bei diesen Tuben sind daher Seitenöffnungen unbekannt und unter bisheriger Praxis auch nicht zweckmäßig.

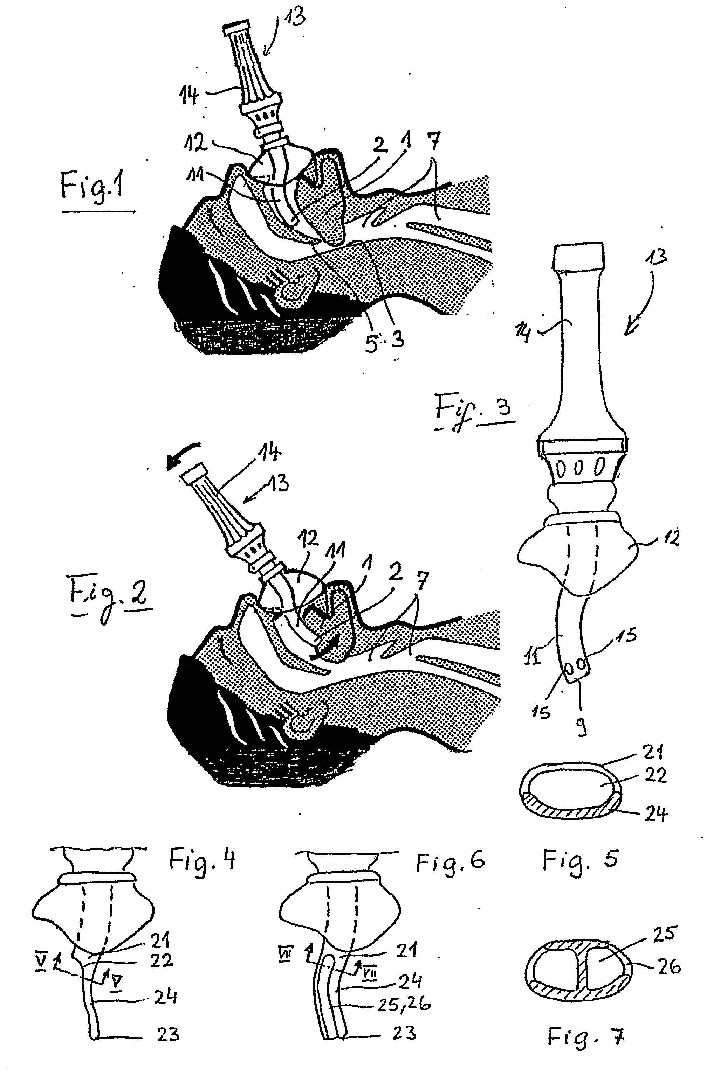

Es ist ein Atemspendehilfsgerät bekannt (US-A 4 535 765), dessen in die Mundhöhle einzusetzender Teil im Anschluß an eine zungenabgewandte, nicht weit hinter der Zahnebene liegenden Öffnung einen schuhlöffelartig geformten Abschnitt aufweist, der nach hinten, unten gekrümmt ist und dazu bestimmt ist, die Zunge herabzudrücken. Die Länge dieses Abschnitts ist unbestimmt, weil der Lippenschild in Längsrichtung verschiebbar ist, so daß die Länge des in die Mundhöhle einzusetzenden Teils je nach den Vorstellungen des Atemhelfers unterschiedlich eingestellt werden kann. Es geht auch nicht daraus hervor, wie man sich die korrekte Stellung dieses Abschnitts über der Zunge vorzustellen hat. Anzunehmen ist, daß er so lang und so stark gekrümmt sein soll, daß sein Ende sich im Rachenbereich hinter die Zunge setzt und sie dadurch an der Anlage an der hinteren Rachenwand hindern soll. Dies bedeutet, daß das Gerät ähnlich einem Pharyngealtubus erst hinter dem Gaumensegel endet und länger als daumenlang ist. Die Annahme wird durch Fig. 2 der genannten Schrift gestützt. Diese zeigt ein Gerät, das weit über den Beginn des Gaumensegels hinausragt, fast bis in den Rachen. Dies geht daraus hervor, daß erstens der harte Gaumen (knöcherner Anteil des Gaumens) beim Menschen horizontal verläuft und erst das sich dorsal anschließende Gaumensegel (weicher Gaumen) in je nach Funktionszustand mehr oder weniger scharfem Winkel nach caudal abgewinkelt ist, und daß zweitens die Länge des harten Gaumens maximal dem Abstand der Oberkieferzahnwurzeln von den Unterkieferzahnwurzeln bei leicht geöffnetem Mund entspricht. Damit kann die strich punktierte Linie, die in der genannten Figur zur Begrenzung der Mundhöhle dient, allenfalls in dem Bereich, in dem sie von der Schraffur begleitet wird, den harten Gaumen darstellen; der Rest entspricht dem Gaumensegel. Ein weiteres Indiz ist drittens die Kontur des Unterkiefers: Das dorsale Ende ist andeutungsweise nach cranial abgebogen, so daß der Zeichner hier den Kieferwinkel angenommen haben dürfte. Die Kieferwinkel befindet sich beim Menschen in einer Entfernung vom Kinn, die im Mund der Entfernung zum Rachen entspricht.

Bei einem anderen bekannten Atemspendehilfsgerät (US-A 3 124 124) soll der in die Mundhöhle einzusetzende Tubus in der Art von Pharyngealtuben eine direkte Verbindung zwischen der Maske und dem Rachen des Patienten sicherstellen.

Der Erfindung liegt die Aufgabe zugrunde, ein Atemspendehilfsgerät der eingangs genannten Art zu schaffen, bei dem die Freihaltung der Atemwege ohne Verletzungs- und Nebenwirkungsgefahr auch bei Anwendung durch Laienhelfer entscheidend erleichtert wird.

Die Erfindung geht von der Erkenntnis aus, daß es mittels des daumenlangen Tubus oder eines in der Form vergleichbaren Geräts möglich ist, die gegebenenfalls in den Rachenraum zurückgefallene Zunge durch eine besondere Bewegung aus dem Atemweg zu entfernen. Dies geschieht dadurch, daß der Tubus zunächst in diejenige Position zwischen Gaumen und Zunge vollständig eingeschoben wird, in der die bekannten Geräte statisch verharren. Ausgehend von dieser Grundstellung wird er jedoch mit seinem Ende nach unten (caudal) und vorne (ventral) verschwenkt. Dabei greift sein Ende in die Masse der Zunge ein und zieht sie mit sich nach vorne. Bei bekannten Tuben verbietet sich diese Bewegung, weil ihre Endöffnung dadurch zwangsläufig verschlossen wird.

Die erfindungsgemäße Lösung besteht daher darin, daß das Atemspendehilfsgerät eine zungenabgewandte, beim Vorhebeln der Zunge mit dieser nicht in Verschlußberührung kommende Atemluftöffnung aufweist. Wenn der in den Mund einzuführende Teil des Geräts in seiner Gesamtheit als Tubus ausgeführt ist, ist die Öffnung in der Wand des Tubus angeordnet. Jedoch ist eine tubusförmige Ausbildung dieses Teils des Geräts nicht erforderlich; das Vorhebeln der Zunge kann vielmehr auch mit einem löffel- oder stiel- oder spatelartig ausgebildeten Teil geschehen, wobei die Öffnung an irgendeiner Stelle angeordnet sein kann, die bei der Benutzung des Geräts frei liegt und insbesondere in Entfernung von dem Ende dieses Teils gelegen sein kann. Obwohl der Tubus im Lippenbereich rohrförmig ausgeführt ist, wie es dem engeren Sinn des Wortes entspricht, kann der endnähere Teil auch eine andere Querschnittsgestalt haben, beispielsweise eine H-Form mit offenen Nuten, die sowohl Luftleitquerschnitte als auch seitlich oder zum Gaumen gerichtete, zungenabgewandte Öffnungen bilden. Ferner ergibt sich aus dem Funktionszusammenhang, daß die Endöffnung entfallen kann. Vorausgesetzt wird in jedem Fall, daß das Gerät starr mit einem außerhalb des Patientenmundes gelegenen Teil verbunden ist, mittels dessen der Helfer die Hebelbewegung ausführen kann. Die Ausbildung des Einblasmundstücks wie bei bekannten Geräten bietet sich an.

Zwar ist es bekannt (FR-A 1 515 163), bei einem daumenlangen, vor dem Gaumensegel endenden Atemspendehilfstubus die Atemluftöffnung auf der Zunge abgewandten Seite vorzusehen; dabei ist jedoch der außerhalb des Mundes liegende Teil des Geräts so kurz ausgeführt, daß die Hand daran nicht den Platz findet, der erforderlich wäre, das Gerät entgegen der Rückstellkraft der Zunge im Mund so einzustellen, wie es die Erfindung verlangt.

Die erfinderische Leistung liegt wesentlich in der Auffindung des Verfahrens zum Vorhebeln der Zunge aus einer eventuellen Verschlußlage. Die darin liegende Erfindungshöhe kommt auch dem Gerät zugute, das für die Durchführung diese Verfahrens besonders ausgebildet ist. Insbesondere beschränkt sich die erfinderische Leistung nicht darauf, bei einem daumenlangen Tubus eine Seitenöffnung vorgesehen zu haben.

Die Erfindung wird im folgenden näher unter Bezugnahme auf die Zeichnungen erläutert, die vorteilhafte Ausführungsbeispiele veranschaulicht. Es zeigen:

Fig. 1 eine schematische Querschnittsdarstellung der Atemwege des Patienten, mit dem Atemspendehilfsgerät in der Grundstellung und der Zunge in der Verschlußlage;

Fig. 2 eine der Fig. 1 entsprechende Darstellung der erfindungsgemäßen Anwendung,

Fig. 3 eine Seitenansicht eines Geräts

Fig. 4 eine der Fig. 3 entsprechende Teil-Seitenansicht einer anderen Ausführungsform,

Fig. 5 einen Querschnitt in etwa natürlichem Maßstab gemäß Linie V-V der Fig. 4,

Fig. 6 eine der Fig. 4 entsprechende Teil-Seitenansicht einer weiteren Ausführungsform und

Fig. 7 eine der Fig. 5 entsprechende Querschnittsdarstellung gemäß Linie VII-VII der Fig. 6.

In den Fig. 1 und 2 sind Kopf und Unterkiefer des Patienten in der für die Atemspende üblichen, weil für die Freiheit der Atemwege förderlichsten, Haltung dargestellt: Der Kopf ist in den Nacken überstreckt, der Unterkiefer ist an den Oberkiefer herangeklappt, soweit das Hilfsmittel dies erlaubt, und möglichst weit kinnwärts gezogen. Bei Verwendung eines kurzen Tubus zur Beatmung besteht - wie Fig. 1 veranschaulicht - auch bei korrekter Lagerung des Patienten die Gefahr, daß die Zunge 1 sich an die hintere Rachenwand 3 bzw. an das Gaumensegel 5 anlegt. Die Atemwege 7 sind dann von der Zunge 1 versperrt und die Luft kann dem Bewußtlosen mittels des mit einem Tubus 11, einem Lippenschild bzw. einer Mundabschlußkappe 12 und einem Mundstück 14 versehenen Atemspendehilfsgeräts nicht zugeführt werden.

Fig. 2 zeigt die Stellung des Geräts, nachdem es aus der Grundstellung (Fig. 1) innerhalb der Zeichenebene in Pfeilrichtung geschwenkt wurde. Dabei stützt es sich gegenüber der Schwenkkraft an den Oberkieferzähnen ab. Man erkennt, daß das Ende 2 des Geräts bei dieser Bewegung die Zunge 1 aus der Verschlußlage hebelt, so daß die Atemwege 7 freigemacht werden. Wenn der Tubus 11 eine Endöffnung aufweist, ist sie in dieser Stellung durch das weiche Gewebe der Zunge verschlossen. In diesem Zustand dienen Seitenöffnungen 15 (Fig. 3) in den Wänden des Tubus 11 für die Luftzufuhr.

Wie leicht aus Fig. 2 zu entnehmen ist, ist die konvexe, dem Gaumen zugewandte Seite des Tubus weniger dem Zungengewebe eingedrückt. Es ist deshalb zweckmäßig, daß mindestens eine der Seitenöffnungen 15 in diesem Bereich angeordnet ist.

Damit die beschriebene Funktion zustande kommen kann, muß der auf die Zunge einwirkende Teil des Geräts hinter dem Lippenschild bzw. der Mundabschlußkappe 12 etwa daumenlang sein und vor dem Gaumensegel enden.

In der Ausführung gemäß Fig. 4 ist der tubusförmige Teil 21 des Geräts verkürzt aufgeführt, so daß die Atemluftöffnung 22 fern von seinem Ende 23 liegt. Der dazwischen befindliche, auf die Zunge einwirkende Teil 24 ist im Querschnitt spatel- oder löffelförmig ausgeführt, wie es Fig. 5 darstellt.

In der Ausführung gemäß Fig. 6 endet der rohrförmige Teil 21 gleichfalls in Abstand vom Ende 23, wobei der dazwischenliegende Teil im Querschnitt gemäß Fig. 7 H-förmig ausgebildet ist, so daß als Luftleitquerschnitte Nuten 25 mit Seitenöffnungen 26 gebildet werden. Die besondere Querschnittsform verleiht dem Gerät hohen Biegewiderstand und sichert auch unter ungünstigsten Umständen hinreichend große Luftleitquerschnitte und Übergangsöffnungen.

Die dargestellte Krümmung des auf die Zunge einwirkenden Teils mit zur Zunge hin gewendetem Ende ist für die beschriebenen Zwecke günstig.

## Patentansprüche

1. Atemspendehilfsgerät mit einem in die Mundhöhle zwischen Gaumen und Zunge (1) einzusetzenden, etwa daumenlangen, vor dem Gaumensegel (5) endenden Teil (11), einem damit starr verbundenen, außerhalb des Mundes zu liegen bestimmten Teil (13), mittels dessen die Winkelstellung des in der Mundhöhle befindlichen Teils (11) vom Helfer eingestellt werden kann, und einem die beiden Teile (11, 13) voneinander abgrenzenden Lippenschild oder einer Mundabschlußkappe (12), dadurch gekennzeichnet, daß der in die Mundhöhle einzusetzende Teil (11) eine zungenabgewandte, beim Vorhebeln der Zunge mit dieser nicht in Verschlußberührung kommende Atemluftöffnung (15, 22, 26) aufweist.

2. Atemspendehilfsgerät nach Anspruch 1, dadurch gekennzeichnet, daß es als Tubus (11) ausgeführt ist, in dessen Wand die Öffnung (15) angeordnet ist.

3. Atemspendehilfsgerät nach Anspruch 1, dadurch gekennzeichnet, daß die Öffnung (22,26) in Abstand von dem Ende des Geräts angeordnet ist bzw. beginnt und daß der die Öffnung (22,26) bzw. deren Beginn überragende Teil (24) löffel- oder spatelartig ausgebildet ist.

4. Atemspendehilfsgerät nach Anspruch 3 dadurch gekennzeichnet, daß der löffel- oder spatelartige Teil (24) einen H-förmigen Querschnitt aufweist.

## Claims

1. A resuscitation aid having a part (11) to be inserted into the oral cavity between the palate and the tongue (1), which part (11) is of approximately thumb length and ends before the soft palate (5), a part (13) which is rigidly connected to the part (11) and intended to be situated outside the mouth, by means of which the angular position of the part (11) disposed in the oral cavity can be adjusted by the helper, and a lip shield or a mouth closure cap (12) delimiting the two parts (11, 13) from one another, characterised in that the part (11) to be inserted into the oral cavity has a respiratory air opening (15, 22, 26) which is remote from the tongue and which does not come into sealing contact with the tongue during manipulation thereof.

2. A resuscitation aid according to Claim 1, characterised in that it is in the form of a tube (11), the opening (15) being provided in the wall thereof.

3. A resuscitation aid according to Claim 1, characterised in that the opening (22, 26) is disposed or begins at a distance from the end of the appliance, and in that a part (24) overlapping the opening (22, 26) or its beginning is of spoon or spatula-like shape.

4. A resuscitation aid according to Claim 3, characterised in that the spoon or spatula-like part (24) is of H-shaped cross-section.

## Revendications

1. Appareil d'assistance respiratoire, comprenant une partie (11) destinée à être insérée dans la cavité buccale entre le palais et la langue (1), ayant à peu près la longueur du pouce et se terminant en avant du voile du palais (5), une partie (13) qui est fixée rigidement à cette première partie, est destinée à se trouver hors de la bouche et au moyen de laquelle la position angulaire de la partie (11) qui se trouve dans la cavité buccale peut être réglée par le secouriste, et une plaque labiale ou une calotte de fermeture de la bouche (12) qui délimite ces deux parties l'une par rapport à l'autre, caractérisé en ce que la partie (11) destinée à être insérée dans la cavité buccale présente, du côté opposé à la langue, un orifice d'air respiratoire (15, 22, 26) qui n'entre pas en contact obturant avec la langue lorsque celle-ci est soulevée en avant.

2. Appareil d'assistance respiratoire selon la revendication 1, caractérisé en ce qu'il est réalisé sous forme de tube (11) dans la paroi duquel est disposé l'orifice (15).

3. Appareil d'assistance respiratoire selon la revendication 1, caractérisé en ce que l'orifice (22, 26) est disposé ou débute à distance de l'extrémité de l'appareil et en ce que la partie (24) située au-delé de l'orifice (22, 26) ou du début de celui-ci est réalisée en forme de cuiller ou d'abaisse-langue.

4. Appareil d'assistance respiratoire selon la revendication 3, caractérisé en ce que la partie en forme de cuiller ou d'abaisse-langue (24) présente une section transversale en H.

Fig.1

Fig.2

Fig.3

Fig.4

Fig.5

Fig.6

Fig.7